# EUROPEAN PATENT APPLICATION

(11) **EP 4 173 575 A1**
(43) Date of publication of application: **03.05.2023**
(21) Application number: 21858148.6
(22) Date of filing: 30.07.2021
(51) Int. Cl.: A61B 10/06

(54) **BIOPSY FORCEPS**

(30) Priority: 20.08.2020 JP 2020139123
(71) Applicant: Biomedical Solutions Inc., Tokyo 103-0023 (JP)
(72) Inventor: ISHIDA, Hidekazu, Nishinomiya-shi, Hyogo 662-0832 (JP); ISHIDA, Hiroki, Tokyo 103-0023 (JP); MIKATA, Mai, Tokyo 103-0023 (JP)
(74) Representative: Taruttis, Tilman
(86) International application number: PCT/JP2021/028292
(87) International publication number: WO 2022/039000

(57) **Abstract**

A biopsy forceps includes: a forceps part having a forceps cup that may open and close; a bending part that is configured to be bendable and includes the forceps part at the front end thereof; an operation part for operating opening and closing of the forceps cup and bending of the bending part; a shaft part coupled to the bending part and the operation part; a first operating member that is inserted through the shaft part, the first operating member being for opening and closing the forceps cup in conjunction with the operation of the operation part; a second operating member that is inserted through the shaft part, the second operating member being for bending the bending part in conjunction with the operation of the operation part; and a contrast agent supply tube that is inserted through the shaft part, the contrast agent supply tube having a distal end open to the forceps part and a proximal end open to the operation part.

## Description

### TECHNICAL FIELD

The present invention relates to biopsy forceps.

### BACKGROUND ART

Conventionally, biopsy forceps have been known, in which a scissors-shaped forceps cup is provided on a distal side of a shaft portion having flexibility and an operation portion for the forceps cup is provided on a proximal side of the shaft portion (see Patent Document 1).
Patent Document 1: Japanese Unexamined Patent Application, Publication No.2005-218486

### DISCLOSURE OF THE INVENTION

### Problems to be Solved by the Invention

In a case where the forceps cup of the biopsy forceps is guided to an examination area, a practitioner performs such a process with reference to an X-ray transmission image obtained using a contrast agent. However, there is a problem that even by the process with reference to the X-ray transmission image obtained using the contrast agent, it is difficult, depending on the morphology of a patient's heart, to cause a tip end of the forceps cup to point in an intended direction to collect a tissue from an intended area.

An object of the present invention is to provide biopsy forceps capable of causing a tip end of a forceps cup to point in an intended direction.

### Means for Solving the Problems

A first aspect of the invention relates to biopsy forceps including a forceps portion having an openable forceps cup, a bendable portion that is provided with the forceps portion at a tip end and is bendable, an operation portion that is operable to open and close the forceps cup and bend the bendable portion, a shaft portion coupled to the bendable portion and the operation portion, a first operation member that is inserted into the shaft portion and opens or closes the forceps cup in conjunction with operation of the operation portion, a second operation member that is inserted into the shaft portion and bends the bendable portion in conjunction with operation of the operation portion, and a contrast agent supply pipe that is inserted into the shaft portion and has a distal-side end portion opening at the forceps portion and a proximal-side end portion opening at the operation portion.

In the above-described aspect of the invention, the distal-side end portion of the contrast agent supply pipe may open at a position not interfering with the forceps cup of the forceps portion.

In the above-described aspect of the invention, the bendable portion may include a plurality of movable pieces that moves in conjunction with the second operation member, the operation portion may include an operation member that is operable to move the second operation member forward or backward, and the plurality of movable pieces may bring the bendable portion into a bent state or a straight state in conjunction with forward/backward movement of the second operation member by operation of the operation member.

### Effects of the Invention

According to the biopsy forceps of the present invention, the tip end of the forceps cup can point in the intended direction.

### BRIEF DESCRIPTION OF THE DRAWINGS

FIG. 1 is a view showing an entire configuration of biopsy forceps 1 of an embodiment;
FIG. 2A is a view showing a configuration of a forceps portion 10;
FIG. 2B is a view showing the configuration of the forceps portion 10;
FIG. 3A is a view showing a configuration of a bendable portion 20;
FIG. 3B is a view showing the configuration of the bendable portion 20;
FIG. 3C is a view showing the configuration of the bendable portion 20;
FIG. 3D is a view showing the configuration of the bendable portion 20;
FIG. 4 is an exploded view showing configurations of a dial 303 of an operation portion 30 and the periphery thereof;
FIG. 5 is a sectional view along an I-I line of FIG. 1; and
FIG. 6 is a view for describing a state when a tissue is collected from a heart 60 by the biopsy forceps 1.

### PREFERRED MODE FOR CARRYING OUT THE INVENTION

Hereinafter, an embodiment of the present invention will be described. Note that the drawings attached to the present specification are all schematic views, and for the sake of easy understanding, etc., the shape, scale, aspect ratio, etc. of each component are changed or exaggerated from actual shape, scale, aspect ratio, etc. Moreover, in the drawings, hatching for showing the section of each member is omitted as necessary. In the present specification, etc., terms specifying shapes, geometric conditions, and the degrees thereof, such as terms "parallel", "perpendicular", and "direction", include not only exact meanings of these terms, but also ranges which can be taken as substantially parallel, substantially perpendicular, etc. and ranges which can be taken as being substantially in intended directions.

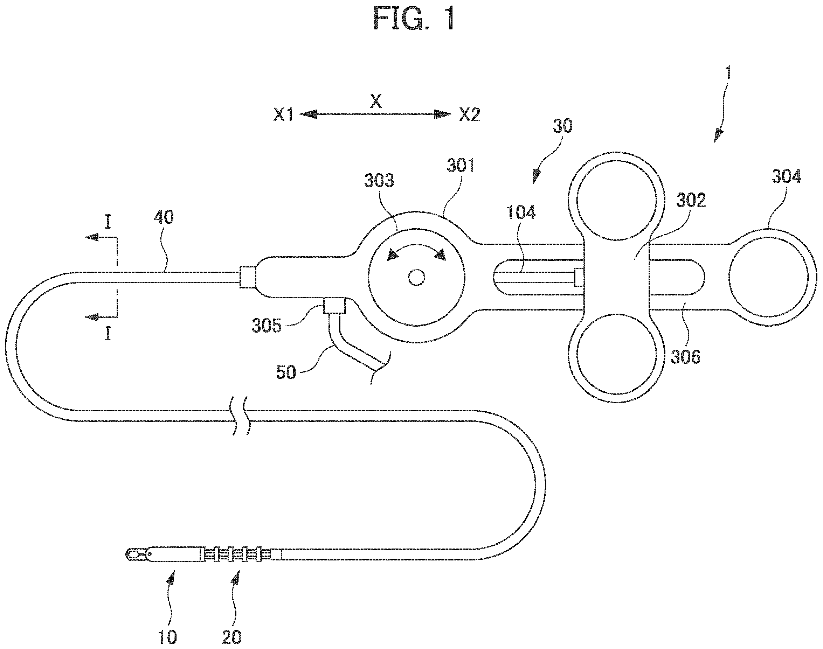

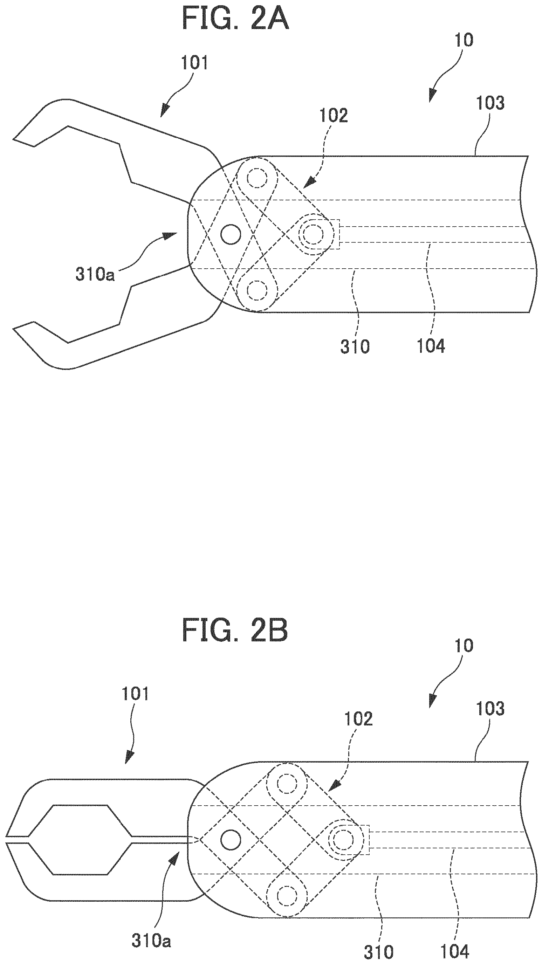

FIG. 1 is a view showing an entire configuration of biopsy forceps 1 of the present embodiment. FIGS. 2A and 2B are views showing a configuration of a forceps portion 10. FIG. 2A shows the forceps portion 10 when a forceps cup 101 is in an open state. FIG. 2B shows the forceps portion 10 when the forceps cup 101 is in a closed state. Note that in the present specification and the drawings, in an X-direction which is a longitudinal direction (or an axial direction) of the biopsy forceps 1, a direction toward a distal side (a tip end side) farther from a practitioner is an X1-direction and a direction toward a proximal side (a base end side) closer to the practitioner is an X2-direction, as shown in FIG. 1. Moreover, in the present specification, "... direction" will be also referred to as "... side", as necessary.

As shown in FIG. 1, the biopsy forceps 1 of the present embodiment include the forceps portion 10, a bendable portion 20, an operation portion 30, and a shaft portion 40.

### (Forceps Portion 10)

The forceps portion 10 is a component provided closest to the distal side (the X1-side) of the biopsy forceps 1. As shown in FIGS. 2A and 2B, the forceps portion 10 includes the forceps cup 101, a link mechanism 102, and a holding portion 103. The forceps cup 101 is a scissors-shaped member that collects a tissue of an examination area, and is openable. The "scissors shape" includes, for example, a form in which a pair of hook-shaped members, cutting members, etc. opens or closes relative to each other. The pantagraph-shaped link mechanism 102 is coupled to the forceps cup 101. The link mechanism 102 is a mechanism that opens or closes the scissors-shaped forceps cup 101 in conjunction with forward/backward movement of a forceps operation wire 104 (described later). A distal-side end portion of the forceps operation wire 104 (described later) is joined to the link mechanism 102. The holding portion 103 is a substantially-tubular component that movably supports the forceps cup 101 and the link mechanism 102.

As shown in FIGS. 2A and 2B, a distal-side (Xl-side) end portion 310a of a liquid feeding tube 310 (described later) opens at the holding portion 103. Thus, the biopsy forceps 1 can release a contrast agent from the tip end of the forceps portion 10. In the present embodiment, the distal-side end portion 310a of the liquid feeding tube 310 opens at a position not interfering (not overlapping) with the forceps cup 101. With this configuration, interference of the end portion 310a of the liquid feeding tube 310 with operation of the forceps cup 101 and contact of the end portion 310a with the collected tissue can be reduced.

Note that the same configuration as that of a forceps portion of general conventional biopsy forceps is applicable to the forceps portion 10 of the biopsy forceps 1 of the present embodiment. That is, the link mechanism 102 of the forceps portion 10 is not limited to the example described in the present embodiment, and various link mechanisms are applicable. The forceps cup 101 is not limited to the example of the present embodiment as long as the tissue of the examination area can be collected, and a forceps cup in any shape may be employed.

The forceps operation wire (a first operation member) 104 is an elongated member that is inserted into the shaft portion 40 (described later) and opens or closes the forceps cup 101 in conjunction with operation of the operation portion 30. The forceps operation wire 104 is, for example, made of stainless steel (SUS). As described above, the distal-side (Xl-side) end portion of the forceps operation wire 104 is coupled to an end portion of the link mechanism 102. A proximal-side (X2-side) end portion of the forceps operation wire 104 penetrates the bendable portion 20 and the shaft portion 40, and is joined to a slider 302 (described later) of the operation portion 30.

As described later, when the practitioner operates the slider 302 of the operation portion 30 to move the forceps operation wire 104 forward to the distal side, the forceps cup 101 is brought into the open state as shown in FIG. 2A. When the practitioner operates the slider 302 of the operation portion 30 to move the forceps operation wire 104 backward to the proximal side, the forceps cup 101 is brought into the closed state as shown in FIG. 2B. The practitioner inserts the biopsy forceps 1 into a body and opens or closes the forceps cup 101 with the forceps portion 10 positioned close to the examination area, and in this manner, can collect the tissue of the examination area.

### (Bendable Portion 20)

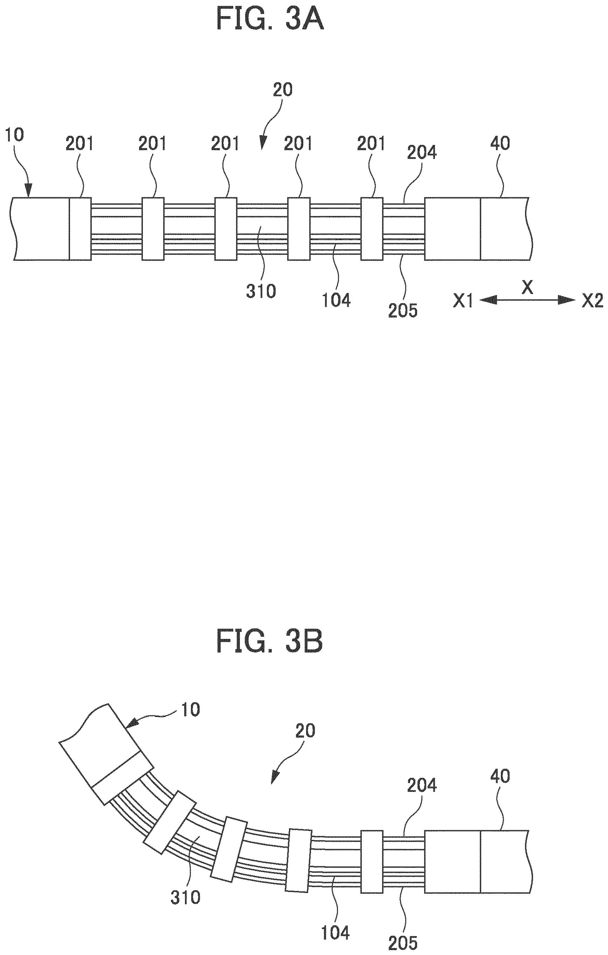

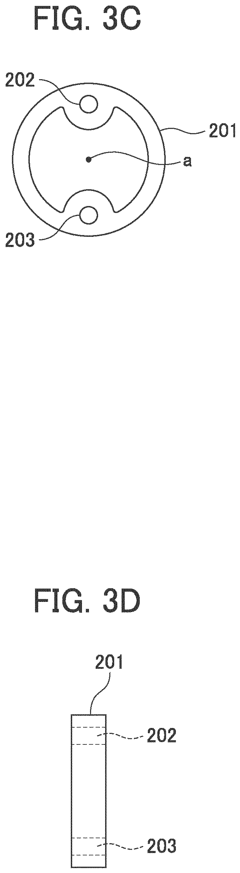

FIGS. 3A to 3D are views showing a configuration of the bendable portion 20. FIG. 3A is the view showing the bendable portion 20 in a straight state. FIG. 3B is the view showing the bendable portion 20 in a bent state. FIG. 3C is the view showing a movable piece 201 from the axial direction (the X-direction). FIG. 3D is the view showing the movable piece 201 from a direction perpendicular to the axial direction.

The bendable portion 20 is a portion that is bendable with a later-described mechanism and is displaceable to the bent state or the straight state. As shown in FIG. 3A, the bendable portion 20 includes five movable pieces 201 arrayed at equal intervals along the axial direction (the X-direction). Of the five movable pieces 201, the movable piece (hereinafter also referred to as a "leading movable piece") 201 positioned closest to the distal side (the X1-side) is joined to the forceps portion 10. Note that the present embodiment describes the example where the five movable pieces 201 of the bendable portion 20 are arrayed, but the number of movable pieces 201 is not particularly limited and may be six or more or four or less.

As shown in FIGS. 3C and 3D, the movable piece 201 is a substantially ring-shaped component, and on an inner diameter side, is provided with a first through-hole 202 and a second through-hole 203. As shown in FIG. 3C, the first through-hole 202 and the second through-hole 203 are provided at positions facing each other in a radial direction about the center axis a of the movable piece 201. The first through-hole 202 is a circular hole into which a bending operation wire 204 is to be inserted. A distal-side (Xl-side) end portion of the bending operation wire 204 is joined to the first through-hole 202 of the leading movable piece 201. In the other movable pieces 201, the bending operation wire 204 is not joined to the first through-holes 202. That is, the bending operation wire 204 is movably inserted into the first through-holes 202 of the other movable pieces 201. Note that although not shown in FIGS. 3A to 3D, the forceps operation wire 104 and the liquid feeding tube 310 are inserted into the movable pieces 201, as described later.

The bending operation wire (a second operation member) 204 is a member that is inserted into the shaft portion 40 and brings the bendable portion 20 into the bent state or the straight state in conjunction with operation of the operation portion 30. The bending operation wire 204 is, for example, made of stainless steel (SUS). The distal-side (Xl-side) end portion of the bending operation wire 204 is, as described above, joined to the first through-hole 202 of the leading movable piece 201. A proximal-side (X2-side) end portion of the bending operation wire 204 penetrates the shaft portion 40, and is joined to the dial 303 (described later) of the operation portion 30.

The second through-hole 203 is a circular hole into which an elastic wire 205 is to be inserted. The elastic wire 205 is a member made of a metal material having a high elasticity. For example, a hyperelastic material such as shape-memory alloy can be used for the elastic wire 205. The elastic wire 205 of the present embodiment is normally in a straight state, and when external force acts thereon, deforms in the direction of such external force. The elastic wire 205 has such properties that when the external force no longer acts on the elastic wire 205, the elastic wire 205 returns to the original straight state. Note that the elastic wire 205 of the present embodiment is formed of a member having a circular section, but may be formed of a member having a rectangular section, for example. The elastic wire 205 is joined to the second through-holes 203 of all the movable pieces 201.

As described later, when the practitioner operates the dial 303 of the operation portion 30 to move the bending operation wire 204 forward to the distal side, the bendable portion 20 is brought into the straight state by elastic force of the elastic wire 205 as shown in FIG. 3A. When the practitioner operates the dial 303 of the operation portion 30 to move the bending operation wire 204 backward to the proximal side, the bendable portion 20 deforms in the direction of the first through-hole 202 against the elastic force of the elastic wire 205 and is brought into the bent state as shown in FIG. 3B. From this state, when the practitioner further operates the dial 303 of the operation portion 30 to move the bending operation wire 204 forward to the distal side, the bendable portion 20 is brought into the straight state shown in FIG. 3A by the elastic force (restoring force) of the elastic wire 205.

### (Operation Portion 30)

The operation portion 30 is a portion that is operable to open and close the forceps cup 101 (the forceps portion 10) and bend the bendable portion 20. As shown in FIG. 1, the operation portion 30 includes an operation portion body 301, the slider 302, the dial (an operation member) 303, a holding ring 304, and a liquid feeding ferrule 305. The operation portion body 301 is a portion to be gripped by the practitioner. The operation portion body 301 is, for example, made of plastic, metal, etc. A guide rail 306 that guides movement of the slider 302 in the X-direction is provided at the operation portion body 301. The slider 302 is a member to be operated when the practitioner opens or closes the forceps portion 10 (the forceps cup 101), and is attached to the guide rail 306. The slider 302 is movable in the X-direction (the X1- and X2-directions) along the guide rail 306. The forceps operation wire 104 is joined to a distal-side (Xl-side) end portion of the slider 302. Although not shown in the figure, the forceps operation wire 104 is inserted into the operation portion body 301 at such a position that the forceps operation wire 104 does not interfere with a drum 307 (described later). The holding ring 304 is provided at a proximal-side (X2-side) end portion of the operation portion body 301.

The practitioner grips the operation portion 30 by inserting one's thumb into the holding ring 304 of the operation portion 30 and inserting one's index and middle fingers into two rings of the slider 302, and in this state, can move the slider 302 along the X-direction relative to a reference point which is the holding ring 304. When the practitioner moves the slider 302 to the X1-side to move the forceps operation wire 104 forward to the distal side, the forceps cup 101 (the forceps portion 10) is brought into the open state. When the practitioner moves the slider 302 to the X2-side to move the forceps operation wire 104 backward to the proximal side, the forceps cup 101 is brought into the closed state.

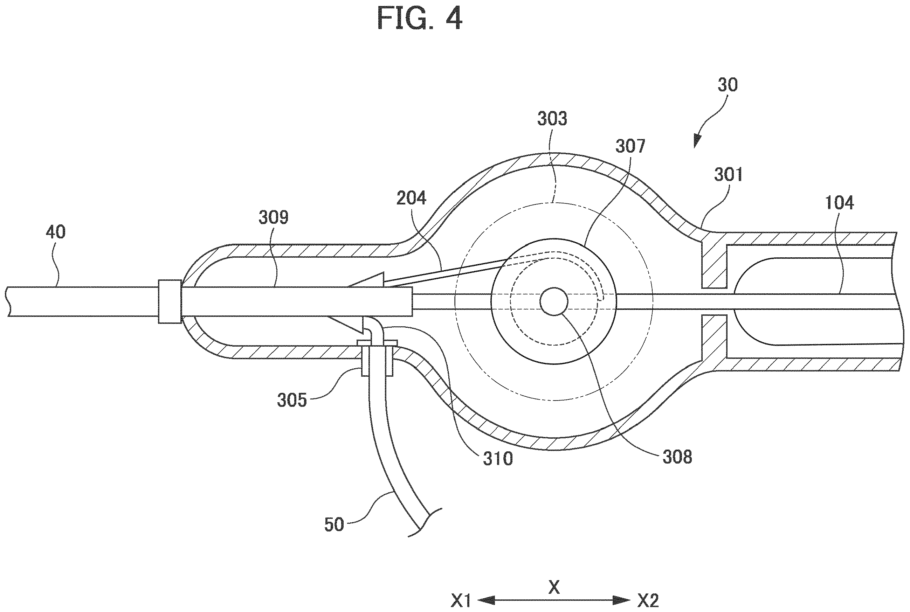

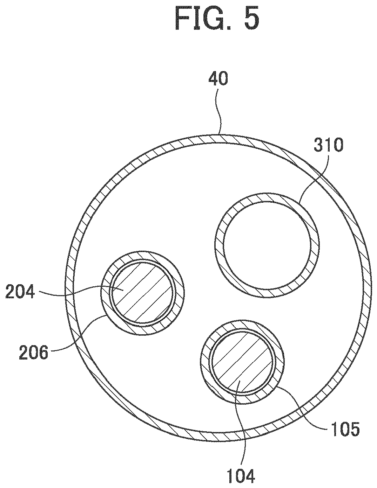

FIG. 4 is an exploded view showing configurations of the dial 303 of the operation portion 30 and the periphery thereof. FIG. 5 is a sectional view along an I-I line of FIG. 1. As shown in FIG. 4, the dial 303 is, in the operation portion body 301, coupled to the drum 307 through a shaft 308. As shown in FIG. 1, the dial 303 is provided on a near side with respect to the operation portion body 301. The drum 307 is a component that winds or unwinds the bending operation wire 204. The proximal-side (X2-side) end portion of the bending operation wire 204 is joined to the drum 307. When the practitioner rotates the dial 303, drive force obtained by such rotation is transmitted to the drum 307 through the shaft 308. According to the direction of the drive force transmitted through the shaft 308, the drum 307 rotates clockwise or counterclockwise. According to the direction of rotation of the drum 307, the bending operation wire 204 is wound round or unwound from the drum 307.

By rotating the dial 303 of the operation portion 30, the practitioner can displace the bendable portion 20 (see FIG. 1) to the bent state or the straight state. For example, when the practitioner rotates the dial 303 of the operation portion 30 clockwise, the bending operation wire 204 is wound around the drum 307, and moves backward to the proximal side (the X2-side). Accordingly, the bendable portion 20 deforms in the direction of the first through-hole 202 against the elastic force of the elastic wire 205, and is brought into the bent state shown in FIG. 3B. On the other hand, when the practitioner rotates the dial 303 of the operation portion 30 counterclockwise, the bending operation wire 204 is unwound from the drum 307, and moves forward to the distal side (the X1-side). Accordingly, the bendable portion 20 is brought into the straight state shown in FIG. 3A by the elastic force (the restoring force) of the elastic wire 205. Moreover, the practitioner adjusts the amount of operation of the dial 303, thereby bringing the bendable portion 20 into a desired bent state.

Next, configurations of other portions of the operation portion 30 will be described. As shown in FIG. 4, a guide tube 309 is provided at a distal-side (Xl-side) end portion in the operation portion body 301. The guide tube 309 is a tubular component into which the forceps operation wire 104, a forceps operation wire guide coil 105 (described later), the bending operation wire 204, a bending operation wire guide coil 206 (described later), and the liquid feeding tube 310 are to be inserted. A distal-side (Xl-side) end portion of the guide tube 309 is joined to the shaft portion 40 (described later). A proximal-side (X2-side) end portion of the guide tube 309 opens to the drum 307.

The forceps operation wire 104 is inserted into the forceps operation wire guide coil 105 (see FIG. 5). The forceps operation wire guide coil 105 is a tubular member formed of a coil spring (not shown) wound in a spiral shape. Although not shown in the figure, a distal-side (Xl-side) end portion of the forceps operation wire guide coil 105 is joined to the forceps portion 10. The distal-side end portion of the forceps operation wire 104 inserted into the forceps operation wire guide coil 105 is joined to the link mechanism 102 of the forceps portion 10 as shown in FIG. 2A.

The bending operation wire 204 is inserted into the bending operation wire guide coil 206 (see FIG. 5). The bending operation wire guide coil 206 is a tubular member formed of a coil spring (not shown) wound in a spiral shape. Although not shown in the figure, a distal-side (Xl-side) end portion of the bending operation wire guide coil 206 is joined to the bendable portion 20. The distal-side end portion of the bending operation wire 204 inserted into the bending operation wire guide coil 206 is joined to the leading movable piece 201 as shown in FIG. 3A.

The liquid feeding tube (a contrast agent supply pipe) 310 is a tubular member that supplies the contrast agent to the forceps portion 10. The liquid feeding tube 310 is, for example, formed of a PEEK tube. The liquid feeding tube 310 is inserted into the shaft portion 40 (described later). The distal-side (Xl-side) end portion of the liquid feeding tube 310 opens at the forceps portion 10 (the holding portion 103) as shown in FIG. 2A. A proximal-side (X2-side) end portion of the liquid feeding tube 310 is connected to the liquid feeding ferrule 305 as shown in FIG. 4. The liquid feeding ferrule 305 is a component into which a contrast agent supply tube 50 is to be inserted. Note that the liquid feeding ferrule 305 may be configured such that a syringe for supplying the contrast agent is connectable to the liquid feeding ferrule 305.

### (Shaft Portion 40)

The shaft portion 40 is a tubular member formed of a coil spring (not shown) wound in a spiral shape. A distal-side (X1-side) end portion of the shaft portion 40 is connected to the bendable portion 20 as shown in FIG. 1. A proximal-side (X2-side) end portion of the shaft portion 40 is connected to the operation portion 30 as shown in FIG. 4. As shown in FIG. 5, the forceps operation wire guide coil 105 into which the forceps operation wire 104 is inserted, the bending operation wire guide coil 206 into which the bending operation wire 204 is inserted, and the liquid feeding tube 310 are housed in the shaft portion 40. Note that arrangement of each component in the shaft portion 40 in FIG. 5 is one example and the present invention is not limited to this example.

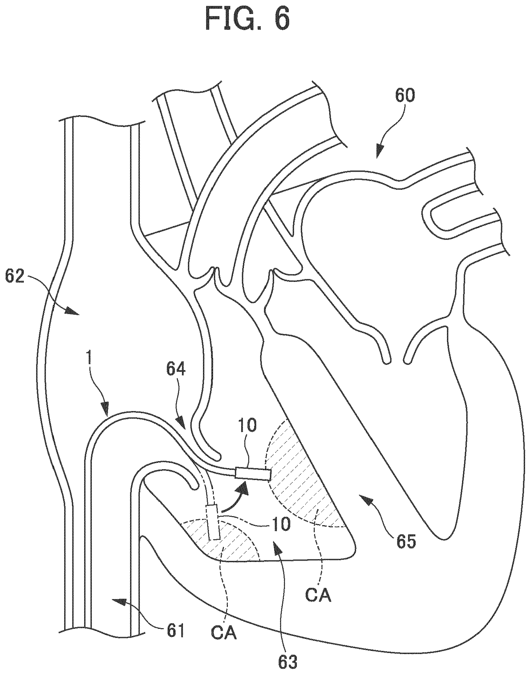

Next, steps of collecting the tissue of the examination area by the biopsy forceps 1 of the present embodiment configured as described above will be described. FIG. 6 is a view for describing a state when the tissue is collected from a heart 60 by the biopsy forceps 1. In FIG. 6, a guide wire, a guiding catheter, etc. are not shown, and the biopsy forceps 1 are simply shown. An example where a tissue in the right ventricle 63 of the heart 60 shown in FIG. 6 is collected will be described herein, and a process until the biopsy forceps 1 are inserted into the guiding catheter will be described with reference to a reference numeral of each area of the heart 60 in FIG. 6 and a process thereafter will be described with reference to FIG. 6.

First, a guide sheath (not shown) is inserted into the right femoral vein 61 of a patient. The guide sheath is a tubular member for guiding, e.g., the guiding catheter into the body. Subsequently, the guide wire and the guiding catheter (not shown) are inserted into the guide sheath, and the guiding catheter is guided to the vicinity of the inferior vena cava by the leading guide wire. Then, the contrast agent is released through a clearance between the guide wire and the guiding catheter, and the guide wire and the guiding catheter are guided to the right atrium 62 while an X-ray transmission image obtained using the contrast agent is being checked. Next, the guide wire is pulled out of the guiding catheter, and an angiographic catheter (not shown) is inserted into the guiding catheter. Then, while the contrast agent is being released from the angiographic catheter, the angiographic catheter is guided to the right ventricle 63 of the heart 60.

Next, the angiographic catheter is pulled out of the guiding catheter, and the biopsy forceps 1 are inserted into the guiding catheter. Then, the biopsy forceps 1 are guided into the right ventricle 63 of the heart 60 as shown in FIG. 6. As shown in FIG. 6, a path from the right femoral vein 61 to the right atrium 62 and a path from the right atrium 62 to the right ventricle 63 curve at an acute angle. Thus, the practitioner operates the dial 303 (see FIG. 1) of the operation portion 30 to bring the bendable portion 20 into the bent state, thereby more smoothly guiding the biopsy forceps 1 to the right ventricle 63.

After having guided the biopsy forceps 1 into the right ventricle 63, the practitioner operates the slider 302 of the operation portion 30 to bring the forceps cup 101 (the forceps portion 10) into the open state. Then, while the contrast agent is being released from the tip end of the forceps portion 10, the forceps portion 10 is guided to the examination area. The practitioner checks an X-ray transmission image obtained using the released contrast agent so that the tip end of the forceps portion 10 can point in an intended direction. For example, as shown in FIG. 6, the contrast agent is released at a position advanced beyond the tricuspid valve 64 (i.e., a position in the right ventricle 63) so that the tip end of the forceps portion 10 can point in the direction of the septum 65 which is the examination area. Note that FIG. 6 schematically shows an area of the contrast agent CA. The practitioner repeats a process of releasing the contrast agent and adjusting the direction of the tip end of the forceps portion 10, thereby accurately guiding the tip end of the forceps portion 10 to the examination area. Once the practitioner determines that the tip end of the forceps portion 10 has reached the examination area, the practitioner operates the slider 302 of the operation portion 30 to bring the forceps cup 101 into the closed state. In this manner, the tissue of the examination area can be collected.

According to the biopsy forceps 1 of the present embodiment as described above, the contrast agent can be released from the tip end of the forceps portion 10, and therefore, the practitioner can cause the tip end of the forceps portion 10 to point in the intended direction while checking the X-ray transmission image obtained using the released contrast agent.

Note that a child's heart has a small muscle thickness, and for this reason, there is a probability that if a tissue is erroneously collected from an unintended area, the heart is pierced with a hole and an accident such as blood leakage is caused. However, according to the biopsy forceps 1 of the present embodiment, the contrast agent can be released from the tip end of the forceps portion 10, and therefore, the practitioner can easily grasp the direction of the tip end of the forceps portion 10 with reference to the X-ray transmission image. Accordingly, the practitioner can avoid an area with a relatively-small muscle thickness and collect a tissue from an area with a relatively-large muscle thickness, and therefore, can avoid the above-described accident. According to the biopsy forceps 1 of the present embodiment, the dial 303 of the operation portion 30 is operated so that the bendable portion 20 joined to the forceps portion 10 can be brought into the bent state or the straight state. Thus, the forceps portion 10 can more smoothly pass through a blood vessel or an organ. Moreover, the amount of rotation of the dial 303 is adjusted so that the forceps portion 10 can point in a desired direction. Thus, the forceps portion 10 can be more accurately guided to the examination area.

In the biopsy forceps 1 of the present embodiment, the distal-side end portion of the liquid feeding tube 310 opens at the position not interfering with the forceps cup 101. Thus, interference of the liquid feeding tube 310 with operation of the forceps cup 101 and contact of the liquid feeding tube 310 with the collected tissue can be reduced. Moreover, the contrast agent can be released in the direction in which the forceps portion 10 points.

In the biopsy forceps 1 of the present embodiment, the bendable portion 20 includes the plurality of movable pieces 201 that move in conjunction with the bending operation wire 204. The plurality of movable pieces 201 brings the bendable portion 20 into the bent state or the straight state in conjunction with forward/backward movement of the bending operation wire 204 by operation of the dial 303 provided at the operation portion 30. Thus, according to the amount of operation of the dial 303, the bent state of the bendable portion 20 can be more finely adjusted.

The embodiment of the present disclosure has been described above, but the present disclosure is not limited to the above-described embodiment. Various modifications and changes can be made as in later-described modifications, and are included in the technical scope of the present disclosure. The advantageous effects described in the embodiment are merely the list of most suitable effects of the present disclosure, and are not limited to those described in the embodiment. Note that the above-described embodiment and the later-described modifications may be used in combination as necessary, but detailed description thereof will be omitted.

### (Modifications)

The operation portion 30 may include two bending operation wires 204. In this case, each of distal-side end portions of the two bending operation wires 204 is joined to the leading movable piece 201. With this configuration, the plurality of movable pieces 201 can displace the bendable portion 20 to the bent state or the straight state in conjunction with forward/backward movement of the two bending operation wires 204. The plurality of movable pieces 201 may be coupled to each other. The shapes of the plurality of movable pieces 201 may be the same as each other, or be different from each other according to an area in the longitudinal direction. Further, the drum 307 that moves the bending operation wire 204 forward or backward may be provided with a lock mechanism, or the dial 303 may be provided with, e.g., a lever.

In the operation portion 30, the mechanism that moves the bending operation wire 204 forward or backward is not limited to the slider 302 shown in FIG. 4, and for example, may be a grip-shaped mechanism that is fixed on one side and is movable on the other side. The distal-side end portion 310a of the liquid feeding tube 310 may open so as to protrude toward the forceps cup 101.

### EXPLANATION OF REFERENCE NUMERALS

1 biopsy forceps
   10 forceps portion
   20 bendable portion
   30 operation portion
   40 shaft portion
   101 forceps cup
   104 forceps operation wire
   201 movable piece
   204 bending operation wire
   205 elastic wire
   301 operation portion body
   302 slider
   303 dial
   310 liquid feeding tube

## Claims

1. Biopsy forceps comprising:
a forceps portion having an openable forceps cup;
a bendable portion that is provided with the forceps portion at a tip end and is bendable;
an operation portion that is operable to open and close the forceps cup and bend the bendable portion;
a shaft portion coupled to the bendable portion and the operation portion;
a first operation member that is inserted into the shaft portion and opens or closes the forceps cup in conjunction with operation of the operation portion;
a second operation member that is inserted into the shaft portion and bends the bendable portion in conjunction with operation of the operation portion; and
a contrast agent supply pipe that is inserted into the shaft portion and has a distal-side end portion opening at the forceps portion and a proximal-side end portion opening at the operation portion.

2. The biopsy forceps according to claim 1, wherein
the distal-side end portion of the contrast agent supply pipe opens at a position not interfering with the forceps cup of the forceps portion.

3. The biopsy forceps according to claim 1 or 2, wherein
the bendable portion includes a plurality of movable pieces that moves in conjunction with the second operation member,
the operation portion includes an operation member that is operable to move the second operation member forward or backward, and
the plurality of movable pieces brings the bendable portion into a bent state or a straight state in conjunction with forward/backward movement of the second operation member by operation of the operation member.
